# EUROPEAN PATENT APPLICATION

(11) **EP 4 850 599 A1**
(43) Date of publication of application: **09.12.2026**
(21) Application number: 25181067.7
(22) Date of filing: 05.06.2025
(51) Int. Cl.: A61B 5/08, A61B 5/16, A61B 5/00

(54) **VOC ANALYSIS**

(71) Applicant: Koninklijke Philips N.V., 5656 AE Eindhoven (NL)
(72) Inventor: FARLEY, Daniel Philip, Eindhoven (NL); RMAILE, Amir Hussein, Eindhoven (NL); BALTAZAR DOS SANTOS, Nuno Filipe, Eindhoven (NL); IMMINK, Albert Hendrik Jan, Eindhoven (NL)
(74) Representative: Philips Intellectual Property & Standards

(57) **Abstract**

A method and system for determining clinical indicators based on volatile organic compound (VOC) measurements while accounting for a subject's emotional state. A VOC measurement dataset is obtained from body-produced substances of the subject. An emotional state indicator is also obtained. An analyzer applies one or more pre-determined analysis models to the VOC dataset to output clinical indicators. The method of analysis is configurable and is configured based on the subject's emotional state. This allows for calibration of the VOC analysis and resulting clinical indicators based on factors like stress levels that can affect VOC profiles. The system may be integrated into a personal care device with VOC sensors for non-invasive measurements during normal use.

## Description

### FIELD OF THE INVENTION

The present invention relates to analyzing volatile organic compounds (VOCs) in non-invasively sampled body-produced substances, and in particular, to systems and methods for determining one or more clinical indicators from VOC measurements.

### BACKGROUND OF THE INVENTION

It is known to analyze concentrations of volatile organic compounds (VOCs) in exhaled breath as a means of diagnosing pathological conditions. VOC biomarkers have been associated with a wide range of diseases, including asthma, cancer, kidney disease, liver disease, chronic obstructive pulmonary disease (COPD), cystic fibrosis, and diabetes.

Thus, the detection and analysis of VOCs in bodily liquids and gases, or other body-produced substances, presents opportunities for convenient and non-invasive detection of certain biomarkers and/or clinical indicators.

### SUMMARY OF THE INVENTION

The invention is defined by the claims.

It has been the recognition of the inventors that one significant challenge is the potential influence of the emotional state of a subject on measured VOC levels. Stress and other emotional factors can alter metabolic processes and affect the production and release of certain VOCs. This variability introduces uncertainty in using VOC profiles as reliable indicators of health status or disease.

Emotional stress affects autonomic nervous system activity, influencing physiological parameters including heart rate, blood pressure, blood glucose level, body temperature, respiration rate, oxygen saturation, salivary secretion, and sweat production.

This phenomenon is well known in the medical field, where patient anxiety can cause a consistent discrepancy in the measured value of physiological parameters, such as blood pressure, at home as compared with in a clinical setting. This is known as the "white coat effect" or "white coat syndrome" and occurs in up to 35% of individuals and may result in transient elevations in blood pressure of approximately 20/10 mmHg.

It has been observed that emotional stress can modulate the concentration of VOCs exhaled in breath and secreted through the skin. The response of VOC concentration to stress can be rapid, introducing potential variability in measurements taken for diagnostic purposes. Consequently, the emotional state of a subject at the time of VOC sampling may influence disease classification and monitoring outcomes.

In view of the recognition of this problem, the inventors propose herein methods and systems aimed at reducing the impact of emotional state associated VOC variability upon clinical determinations. In particular embodiments disclosed herein propose methods for adapting or configuring analysis of VOCs in dependence upon a subject's emotional state.

An aspect of the invention is a processing device comprising one or more processors configured to perform a method for determining at least one clinical indicator for a subject based on volatile organic compound (VOC) measurements. The method includes obtaining a VOC measurement dataset for the subject indicative of measurement of one or more VOCs in one or more body-produced substances of the subject, obtaining an emotional state indicator for the subject indicative of at least one characteristic of an emotional state of the subject, and applying an analyzer configured to perform analysis of the VOC measurement dataset using one or more pre-determined analysis models and output at least one clinical indicator responsive thereto. A method of analysis applied by the analyzer using the one or more models is configurable, and the method of analysis is configured in dependence upon the emotional state of the subject.

By configuring the analysis method according to the emotional state, the system can account for variations in VOC profiles that may be influenced by stress or other emotional factors.

The VOC measurement dataset may be a non-invasively obtained VOC measurement dataset.

By way of non-limiting example, the one or more body-produced substances may include any one or more of: exhaled breath, sweat, saliva, oral gases, and sebum. In principle, embodiments of the invention are also applicable more broadly to other body-produced substances such as urine. Preferably the body-produced substances are non-invasively expressed, i.e. non-invasively sampleable.

In some embodiments, the clinical indicator may comprise a predicted diagnostic indicator for the subject. Additionally or alternatively, in some embodiments, the clinical indicator may comprise a predicted value of one or more biomarkers for the subject.

The method comprises adjusting or configuring the analysis applied to the measured VOC measurements in dependence upon the emotional state. For example, the analyzer may be understood as being configured to use the one or more pre-determined analysis models to perform mapping of VOC measurement data comprised by the VOC measurement dataset to the at least one clinical indicator, and wherein the calibration of the mapping is configured in dependence upon the emotional state. In other words, a calibration of the analyzer may be understood to be adjusted in dependence upon the emotional state.

Different VOC measurement profiles are associated with each clinical indicator dependent upon the emotional state of the user. The adjusting the calibration of the analysis comprises changing the association between obtained VOC measurements and the clinical indicator, e.g. changing the association between the VOC measurements and a disease state classification.

This can be achieved for example by training or configuring a plurality of different pre-determined analysis models, each configured for application in a different emotional state condition of the subject, or instead by providing an emotional state dependent model wherein one or more parameters of the model can be tuned in dependence upon the subject emotional state.

In this regard, in some embodiments, the analyzer may comprise a bank of different analysis models. The analyzer may be configured to employ a different subset of one or more of the plurality of analysis models depending upon the emotional state. Different models of the bank of models may be configured for application when a subject is in a different emotional state. Thus, the emotional state dependency of the analysis can be built into the training or parameters of each individual model in advance.

In some embodiments, the emotional state indicator comprises an emotional stress measure. By way of non-limiting example, the emotional stress measure may be obtained by measuring any one or more of: a heart rate variability (HRV) measure, a pulse rate variability (PRV) measure, a galvanic skin response (GSR) measure, an electrocardiogram (ECG) measurement, an electroencephalography (EEG) measurement, a body temperature measurement, and a blood pressure measurement. PRV and GSR in particular can be measured using small, compact sensors and so are advantageous for integration in personal devices such as personal care devices or wearable devices.

Additionally or alternatively, in some embodiments, the emotional stress measure may be obtained based on subject self-report data. For example, the obtaining the emotional stress measure may comprise obtaining a self-reported stress score, for example using a user interface.

As will be explained later, in some embodiments, the emotional stress measure may be computed as function of data in the VOC measurement dataset, i.e. based on values of one or more of the VOC measurements.

In some embodiments, the analyzer comprises a higher stress model and lower stress model and is configured to use the higher stress model when the emotional state indicator corresponds to a high stress state and the lower stress model when the emotional state indicator corresponds to a low stress state. This allows for more accurate analysis by using models specifically tailored to different stress levels. By way of example, the higher stress model and lower stress model are pre-determined or configured for determining the clinical indicator when the subject stress level meets different criteria, for example when the stress level is within different ranges. For example, the high and low stress state may be classified based on value ranges of a measured physiological parameter or a subject self-report score.

In some embodiments, the analyzer comprises three or more different analysis models, each corresponding to a different gradation of emotional stress level. Configuring the method of analysis comprises selecting one of the models to employ in dependence upon a gradation of emotional stress level indicated by the emotional state indicator. Here, there are provided a plurality of different stress-dependent models, each applicable responsive to a different level of emotional stress of the subject. This provides finer granularity in adjusting the analysis based on emotional stress levels, potentially improving accuracy across a wider range of emotional states.

In some embodiments, the analyzer is configured to abstain from performing the analysis responsive to the emotional state indicator meeting at least one pre-determined condition (or failing to meet at least one pre-determined condition). For example, in a high stress state, the system may abstain from generating the clinical indicator because reliability would be low in the high stress state. This avoids generating unreliable clinical indicator predictions when the subject emotional state may significantly affect VOC measurements.

In some embodiments, the method may comprise monitoring the emotional state indicator over a period of time and may comprise triggering deployment of the analyzer responsive to detection of the emotional state indicator changing from a state of not meeting the at least one pre-determined condition to a state of meeting the at least one pre-determined condition. This ensures that a prediction of the clinical indicator is automatically determined once the subject emotional stress level is in an appropriate state.

In some embodiments, at least one of the one or more analysis models used by the analyzer comprises one or more model parameters which are configured in dependence upon the emotional state indicator. For example, in some embodiments, at least one of the one or more analysis models comprises a function of the emotional state indicator. For example, in some embodiments, at least one of the one or more analysis models comprises at least one independent variable which is a function of the emotional state indicator. For example, in some embodiments, the emotional state indictor forms an input to at least one of the one or more analysis models. In other words, the analyzer may comprise at least one analysis model for which both emotional state and VOC measurement data form inputs to the model.

Thus, here, a mapping between the VOC measurement profile and the clinical indicator, as represented by one or more parameters of the model, is adjusted depending upon the emotional state, e.g. stress level, of the subject. Thus, instead of providing a plurality of different models, each configured for application when the emotional state indicator of the subject is in a different state, a model is provided wherein emotional state is a variable parameter of the model itself.

In some embodiments, the obtaining of the VOC measurement dataset comprises using a non-invasive VOC sensing subsystem. For example, a non-invasive VOC sensing subsystem may comprise one or more VOC sensors arranged for sensing VOC concentrations in saliva, sweat, exhaled breath and/or sebum. This provides a convenient and user-friendly method for collecting VOC data without the need for invasive procedures, e.g. drawing blood.

In some embodiments, the obtaining of the VOC measurement dataset comprises obtaining the VOC measurement dataset using a VOC sensing subsystem comprising one or more VOC sensors integrated in a personal care device. The personal care device may comprise or consist of an oral care device. This integration allows for routine collection of VOC measurement data during regular personal care activities, potentially increasing the frequency and consistency of measurements.

For example, in some embodiments, a non-invasive VOC sensing subsystem may be integrated in the personal care device comprising one or more VOC sensors arranged to be exposed during normal use of the personal care device to one or more of saliva, sweat and/or exhaled breath.

In some embodiments, the VOC measurement dataset comprises data for each of a plurality of VOC measurement channels.

In some embodiments, each of the plurality of VOC measurement channels may correspond to an output of a respective VOC sensor of a plurality of VOC sensors, for example comprised by a sensing subsystem. Each of the plurality of VOC sensors may be configured with a different sensitivity and/or selectivity to VOC composition in a sampled substance. For example, each of the plurality of VOC sensors may comprise a metal oxide (MOX) sensor configured to generate a respective sensor output and having a different sensitivity or selectivity to VOC composition within a sampled substance. Alternatively, each of the plurality of VOC measurement channels may correspond to a measurement of different individual VOC.

In some embodiments, the VOC measurement dataset comprises data for each of a plurality of VOC measurement channels, and the analysis performed by the analyzer comprises transforming the measured VOC dataset into a reduced dimensionality dataset comprising a reduced number of channels. This may be referred to as a dimensionality reduction operation. The reduced channels might be referred to as 'synthetic channels'.

Each of the channels of the reduced dimensionality dataset may be formed as a function of two or more of the VOC measurement channels. Each of the channels of the reduced dimensionality dataset may be a linear combination of two or more of the VOC measurement channels. For example, the reduced dimensionality dataset may comprise a plurality of synthetic channels, wherein the plurality of synthetic channels is smaller in number than the plurality of VOC measurement channels, and wherein each of the synthetic channels is a linear combination of two or more of the VOC measurement channels.

In some embodiments, the channels of the reduced dimensionality dataset correspond to a pre-determined set of Principal Components of the plurality of VOC measurement channels. This use of Principal Component Analysis can help identify the most significant patterns in the VOC data, potentially improving the accuracy of the clinical indicators derived from the analysis.

For example, calibration or training can be performed in advance wherein Principal Component Analysis can be applied to the VOC measurement channels in different emotional state conditions in order to determine a reduced number of principal components which are each formed from a linear combination of a subset of the VOC measurement channels. During operation of the method, the measured VOC data can be processed to compute the pre-determined principal component channels, and wherein the one or more models of the analyzer are configured to map between these principal component channels and the at least one clinical indicator.

In some embodiments, the analysis performed by the analyzer comprises mapping values for the reduced channels of the reduced dimensionality dataset to at least one clinical indicator.

In some embodiments, the at least one clinical indicator comprises a disease state classifier indicative of a predicted current or future disease state of the subject or a current progress stage of a disease. By way of non-limiting example, the at least one clinical indicator may comprise a classifier of a (predicted) extant or future disease state of the subject with respect to any one or more of the following diseases: chronic kidney disease (CKD), chronic liver disease (CLD), lung cancer, breast cancer, colorectal cancer, cystic fibrosis, diabetes, lung cancer, squamous cell cancer, chronic obstructive pulmonary disease (COPD), COVID-19, asthma, and sleep apnea. This allows the system to provide valuable health information based on the VOC analysis, potentially aiding in early disease detection or monitoring of disease progression.

Additionally or alternatively, in some embodiments, the at least one clinical indicator comprises a predicted concentration of a biomarker. By way of example, in some embodiments, it may comprise a predicted stress-adjusted or stress-calibrated value of a biomarker concentration.

In some embodiments, the obtaining the emotional state indicator comprises sensing at least one biosignal correlated with an emotional stress state of a subject, and applying a stress state classifier to the measured biosignal. By way of non-limiting example, the biosignal may comprise any one or more of: an HRV measure, a PRV measure, a GSR measure, an EEG measurement.

Another aspect of the invention is a system. The system may comprise a personal care device, and a VOC sensing subsystem integrated in the personal care device comprising one or more VOC sensors arranged to be non-invasively exposed to at least one bodily-produced substance during operation of the personal care device. The system further comprises a processing device in accordance with any embodiments described in this disclosure or in accordance with any claim, operatively coupled with the VOC sensing subsystem and configured to obtain the VOC measurement dataset using the VOC sensing subsystem. The processing device may be integrated in the personal care device or may be provided by a separate device, for example by the processing device of a mobile computing device with which the VOC sensing subsystem is communicatively coupled.

In some embodiments, the personal care device may further comprise a personal care subsystem for performing a personal care function. For example, in the case of an oral care device, this may comprise an oral care subsystem, e.g. comprising an oscillation motor for driving oscillation of a brush head.

In some embodiments, the system may further comprise a stress sensing subsystem for measuring an emotional stress state of the subject, and wherein the emotional state indicator comprises the emotional stress state.

In some embodiments, the VOC sensing subsystem comprises at least one VOC sensor integrated in a mouth-receivable portion of the device for sensing concentration of at least one VOC in saliva and/or oral gases of the subject during use of the device. Additionally or alternatively, the VOC sensing subsystem may comprise at least one VOC sensor integrated in a handle portion or in a mouth-receivable portion of the device for sensing concentration of at least one VOC in exhaled breath during use of the personal care device. Additionally or alternatively, the VOC sensing subsystem may comprise at least VOC sensor integrated in a handle portion of the device arranged for sensing concentration of at least one VOC in sweat of the subject during use of the device. This configuration allows for convenient and efficient collection of a VOC measurement dataset during normal use of the personal care device.

Another aspect of the invention is a method for determining at least one clinical indicator for a subject based on volatile organic compound (VOC) measurements. The method comprises obtaining a VOC measurement dataset for the subject indicative of measurement of one or more VOCs in one or more body-produced substances of the subject, obtaining an emotional state indicator for the subject indicative of at least one characteristic of an emotional state of the subject, and applying an analyzer configured to perform analysis of the VOC measurement dataset using one or more pre-determined analysis models and output at least one clinical indicator responsive thereto. A method of analysis applied by the analyzer using the one or more models is configurable, and the method of analysis is configured in dependence upon the emotional state of the subject.

The method may be a computer-implemented method.

Any of the features or options described in this disclosure in relation to embodiments of the processing device aspect of the invention may be applied also to the method aspect of the invention.

A further aspect of the invention is a computer program product comprising computer program code. The computer program code is configured, when run on a processor, to cause the processor to perform a method for determining at least one clinical indicator for a subject based on volatile organic compound (VOC) measurements. The method includes obtaining a VOC measurement dataset for the subject indicative of measurement of one or more VOCs in one or more body-produced substances of the subject, obtaining an emotional state indicator for the subject indicative of at least one characteristic of an emotional state of the subject, and applying an analyzer configured to perform analysis of the VOC measurement dataset using one or more pre-determined analysis models and output at least one clinical indicator responsive thereto. A method of analysis applied by the analyzer using the one or more models is configurable, and the method of analysis is configured in dependence upon the emotional state of the subject.

Any of the features or options described in this disclosure in relation to embodiments of the processing device aspect of the invention may be applied also to the computer program product aspect of the invention.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 outlines steps of an example method in accordance with one or more embodiments of the invention;
FIG. 2 shows components of an example processing device and system, in accordance with one or more embodiments of the invention;
FIG. 3 shows components of a further example processing device and system in accordance with one or more embodiments of the invention, comprising a stress-sensing system for sensing an emotional stress state of a subject;
FIG. 4 illustrates a method in accordance with at least one set of embodiments of the invention in which an analyzer comprises a set of two or more different analysis models for use with different emotional states of the subject;
FIG. 5 illustrates a method in accordance with at least one set of embodiments in which an analyzer is configured to abstain from generating a clinical indicator responsive to an emotional state of the subject meeting at least one pre-determined condition or vice versa; and
FIG. 6 illustrates a method in accordance with at least one set of embodiments in which an analyzer is configured to monitor an emotional state of a subject and abstain from sampling VOC measurements until the emotional state meets a pre-determined condition.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method and system for determining one or more clinical indicators based on volatile organic compound (VOC) measurements while accounting for an emotional state of the subject. A VOC measurement dataset is obtained from body-produced substances of the subject. An emotional state indicator is also obtained. An analyzer applies one or more pre-determined analysis models to the VOC measurement dataset to output a value for one or more clinical indicators. The method of analysis is configured based on an emotional state of the subject. This allows for calibration of the VOC analysis and resulting clinical indicators based on factors such as stress levels, which can affect VOC profiles. The system may be integrated into a personal care device with VOC sensors arranged for making non-invasive measurements during normal use.

Volatile organic compounds (VOCs) are chemical substances that readily evaporate at room temperature. These compounds are produced by various biological processes within the human body. Recent studies have demonstrated that emotional stress can significantly alter the concentration of VOCs in exhaled breath and those excreted from the skin. This phenomenon has been observed and documented in scientific literature. In this regard, reference is made for example to the following papers:
Santos, P., et al (2020). Real Time Mental Stress Detection Through Breath Analysis. In: Camarinha-Matos, L., Farhadi, N., Lopes, F., Pereira, H. (eds) Technological Innovation for Life Improvement. DoCEIS 2020. IFIP Advances in Information and Communication Technology, vol 577. Springer, Cham
Lucchi, G., Crépin, M., Chambaron, S. et al. Effects of psychological stress on the emission of volatile organic compounds from the skin. Sci Rep 14, 7238 (2024).

VOC concentration can change rapidly in response to stress. Accordingly, an individual's emotional state at the time of sample collection may have a substantial impact on the measurement of a VOC panel. Consequently, the emotional state of the subject could influence the classification of an individual's disease state or the prediction of other clinical indicators such as biomarker values.

To address this potential source of variability, methods have been developed in accordance with embodiments of the present invention to assess emotional state prior to collecting VOC samples.

Once the emotional state has been assessed, adjustments can be made to the analysis of the VOC measurements with the aim of improving accuracy in the inference of clinical indicators. By accounting for the influence of emotional state on VOC concentrations, more reliable and consistent results may be obtained.

The term "emotional state" as used herein may refer to a range of psychological conditions experienced by an individual. This may include, but is not limited to, states of stress, anxiety, fear, excitement, or calmness.

Table 1 below shows a non-exhaustive list of example VOCs whose concentrations may be impacted by emotional state as well as the pathologies with which they are potentially associated.

**Table 1**

| **VOC** | **Molecular Weight** | **Associated Pathologies** |
|---|---|---|
| Styrene | 104.15 g/mol | CKD, CLD, Lung cancer |
| 1 2-dimethylbenzene | 106.17 g/mol | Breast cancer, Colorectal cancer |
| Ethanol | 46.07 g/mol | Breast cancer, CKD, CLD, Colorectal cancer, Cystic fibrosis, Diabetes, Lung cancer, Squamous cell cancer |
| 2-propanol | 60.10 g/mol | COPD, Covid 19, Lung cancer |
| 1-propanol | 60.09 g/mol | COPD, Covid 19, Lung cancer |
| 2,3-pentanedione | 100.12 g/mol | Colorectal cancer |
| Benzaldehyde | 106.12 g/mol | Asthma, COPD, Colorectal cancer, Sleep apnoea |
| Ethyl acetate | 88.11 g/mol | Breast cancer, Colorectal cancer, Cystic fibrosis |

Thus, embodiments of the present invention provide a method and system for assessing a subject's emotional state, measuring a volatile organic compound (VOC) profile for the subject, and subsequently correcting the VOC measurement and/or analysis thereof based on the emotional assessment. This approach aims to enhance the accuracy of VOC-based diagnostic and monitoring techniques.

In accordance with one or more sets of embodiments, one or more of the following features may be present.

In some embodiments, the VOC profile is collected via exhaled breath. However, it is also possible to obtain measurements in other body-produced substances such as those exuded by the skin through sweat and sebum.

In some embodiments, the VOC measurements may be performed using a single device that serves an additional function as a personal care device, e.g. an oral healthcare device, e.g. a powered toothbrush. This integration of functions allows for convenient and regular data collection during routine oral care activities.

However, the device could instead be provided as a standalone unit, with a dedicated function of measuring emotional state and VOCs, and optionally also vital signs. This standalone configuration may be suitable for clinical settings or for individuals requiring frequent monitoring.

Alternatively, in some embodiments, the system may comprise a set of connected devices which are together configured to acquire the VOC measurement dataset. For example, these may be arranged to simultaneously stream data to a smartphone application for analysis. This distributed approach potentially allows for specialized sensors and may offer flexibility in terms of device placement and data collection.

Regardless of the specific hardware implementation, at least one set of embodiments of the invention may be understood as comprising four main parts or elements:
1. An emotional state measurement part for assessing an individual's emotional state, for example through the measurement of one or more physiological parameters associated with emotional stress.
2. A VOC measurement part for collecting and analyzing the volatile organic compounds present in one or more body-produced substances of the individual, such as the individual's breath or fluid or sebum emanating from their skin.
3. A VOC emotional state calibration part for adjusting the analysis of the VOC measurements based on the assessed emotional state, for example aiming to account for stress-induced variations in VOC concentrations.
4. A pathology analysis part for interpreting the VOC data in an emotional state calibrated way to provide insights into potential pathological conditions or disease states.

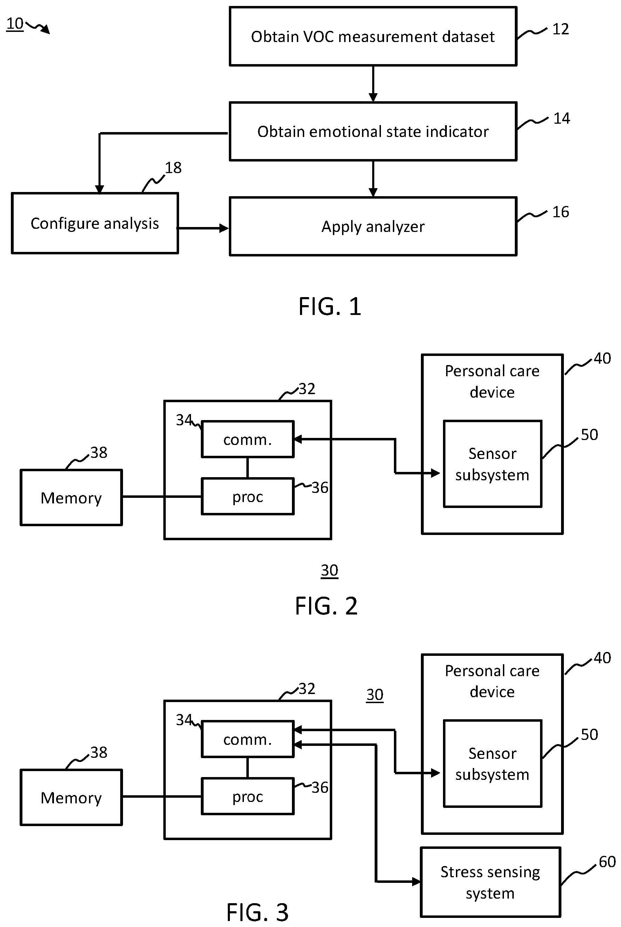

FIG. 1 outlines steps of an example method 10 in accordance with one or more embodiments of the invention. The method 10 is for determining at least one clinical indicator for a subject based on volatile organic compound, VOC, measurements.

The method 10 comprises obtaining 12 a VOC measurement dataset 52 for the subject indicative of measurement of one or more VOCs in one or more body-produced substances of the subject. The one or more body-produced substances may be non-invasively accessible body-produced substances, e.g. excluding blood. By way of non-limiting example, the one or more body-produced substances may include any one or more of: exhaled breath, saliva or oral gases, sweat, or sebum.

The method further comprises obtaining 14 an emotional state indicator for the subject, indicative of at least one characteristic of an emotional state of the subject.

The method further comprises applying 16 an analyzer configured to perform analysis of the VOC measurement dataset using one or more pre-determined analysis models and output at least one clinical indicator responsive thereto. A method or mode of analysis applied by the analyzer using the one or more models is configurable, and the method of analysis is configured 18 in dependence upon the emotional state of the subject.

Another aspect of the invention is a computer program product comprising computer program code configured, when run on a processor, to perform a method in accordance with any embodiment described in this disclosure or in accordance with any claim.

Regarding the clinical indicator, in some embodiments, this may comprise a predicted diagnostic indicator for the subject, for example a disease state classification. Additionally or alternatively, in some embodiments, the clinical indicator may comprise a predicted value of one or more biomarkers for the subject.

FIG. 2 shows components of an example system 30 in accordance with one or more embodiments of the invention. The system comprises a processing device 32 comprising one or more processors 36 configured to perform a method 10 in accordance with any embodiment described in this disclosure or in accordance with any claim. The processing device 32 alone is an aspect of the invention. The system 30 is a further aspect of the invention.

The system 30 may further comprise a memory device 38. The memory device 38 may store computer program code configured when run on the one or more processors 36 of the processing device to cause the one or more processors to perform a method in accordance with any embodiment described in this disclosure or in accordance with any claim.

In the illustrated example, the system 30 further comprises a personal care device 40.

In the illustrated example, the system 30 further comprises a VOC sensing subsystem 50 integrated in the personal care device 40, comprising one or more VOC sensors arranged to be non-invasively exposed to at least one bodily-produced substance, such as at least one body liquid or gas, during operation of the personal care device.

The processing device 32 is operatively coupled with the VOC sensing subsystem 50 and configured to obtain the VOC measurement dataset using the VOC sensing subsystem 50.

Regarding the personal care device 40, in some embodiments this may comprise an oral care device. The oral care device may comprise a powered toothbrush in some embodiments. However, by way of alternative example, the oral care device may comprise a powered flossing device or a powered brushing mouthpiece device. These oral care devices provide convenient platforms for integrating VOC sensors due to their regular contact with the oral cavity.

Instead of an oral care device, the personal care device 40 may comprise or consist of a different type of personal care device. By way of non-limiting example, the personal care device may comprise a grooming device such as a powered shaver device, an epilator device, or an IPL device. These grooming devices offer opportunities for VOC collection from skin surfaces during routine grooming activities.

By way of further non-limiting examples, the personal care device 40 may comprise any one or more of a skin treatment device (such as a cleansing brush or exfoliation tool, a massage device (such as a facial massager or body massager), or a body hair trimming device. In some embodiments, the personal care device may further comprise a multi-function device capable of performing two or more of the aforementioned personal care functions.

The advantage of integrating the sensor system 50 in the personal care device 40 is that it allows for regular, non-invasive collection of VOC data during routine personal care activities. This integration eliminates the need for separate, dedicated VOC sampling sessions, which could be inconvenient or easily forgotten by users. By incorporating VOC sensing into devices that are already part of daily routines, the system can gather data more consistently and frequently.

Furthermore, this integration may improve user compliance with VOC monitoring, as it does not require additional effort or time from the user beyond their normal personal care routines. The familiarity and regular use of personal care devices may also help reduce any anxiety or stress associated with medical testing, potentially leading to more accurate VOC measurements.

In some embodiments, the personal care device 40 may further comprise a personal care subsystem for performing a personal care function. For example, in the case of an oral care device, the personal care subsystem may include a motor for driving a brush head or flossing jet mechanism. For a grooming device, the personal care subsystem may include cutting blades or light-emitting components for hair removal. In a skin treatment device, the personal care subsystem may comprise vibrating or rotating elements for cleansing or exfoliation.

Regarding the VOC sensing subsystem 50, the one or more VOC sensors may be carried by a housing of the personal care device 40 and arranged such that during normal use of the personal care device, for a personal care function, the one or more VOC sensors become exposed to one or more body produced substances, for example one or more bodily liquids or gases.

By way of non-limiting example, in some embodiments, the VOC sensing subsystem 50 comprises at least one VOC sensor integrated in a mouth-receivable portion of the device for sensing concentration of at least one VOC in saliva or oral gases of the subject. For example, the personal care device may comprise an oral care device having a mouth-receivable portion. For example, the mouth-receivable portion may comprise a brush head for a powered toothbrush or a fluid jet head for a powered flossing device.

Additionally or alternatively, in some embodiments, the VOC sensing subsystem 50 may comprise at least one VOC sensor arranged for sensing concentration of at least one VOC in exhaled breath during use of the personal care device. By way of non-limiting example, in some embodiments, the personal care device may comprise a handle portion and wherein the VOC sensing subsystem 50 includes at least one VOC sensor integrated in the handle portion for sensing concentration of at least one VOC in exhaled breath of the subject. This may be advantageous for example in the case of an oral care device having a mouth-receivable portion, and wherein the VOC sensor is arranged facing the mouth of the subject when the mouth-receivable portion is received in the mouth for being exposed to exhaled breath. However, additionally or alternatively, the at least one VOC sensor may be integrated in the mouth-receivable portion itself for sensing exhaled breath.

Additionally or alternatively, in some embodiments, the VOC sensing subsystem 50 may comprise at least one VOC sensor integrated in a handle portion of the device arranged for sensing concentration of at least one VOC in sweat of the subject during use of the device, for example palm sweat.

By way of further illustration FIG. 3 shows a further example system 30 in accordance with one or more embodiments of the invention. The system of FIG. 3 may be the same as the system of FIG. 2 except for the additional inclusion of a stress sensing system 60 for measuring an emotional stress state of the subject. The measured emotional stress state may be used to determine the emotional state indicator. In some examples, the emotional state indicator may consist of the emotional stress state. Alternatively, the emotional state indicator may be determined as a function of the measured emotional stress state.

The stress sensing system may comprise a biosignal measurement device and a processing module (software or hardware, e.g. a processing subroutine comprised by the processing device 32) configured to compute an emotional state indicator using an output from the biosignal measurement device. For example, and by way of non-limiting example, the biosignal measurement device may be configured for measuring any one or more of: heart rate variability (HRV), pulse rate variability (PRV), galvanic skin response (GSR), and electroencephalography (EEG). Optionally, the biosignal measurement device may be integrated in the personal care device or may be provided in a separate device or unit. More details in this regard will follow later.

With regard to the measurement of the one or more VOCs, a variety of sensing techniques and sensor technologies have been developed to enable real-time or near real-time evaluation of VOC constituents in exhaled breath and other body-produced substances. One approach is to use one or more chemical sensors, each configured for detecting a single individual VOC. For example, electrochemical sensors can be designed to detect specific VOCs such as acetone, which is associated with diabetes, or ammonia, which may indicate kidney dysfunction. These sensors typically use selective membranes or electrodes that react specifically with the target VOC, producing a measurable electrical signal.

However, a further approach is to employ one or more chemical sensors each having a broad cross-reactivity to multiple VOCs. Optionally, outputs from such an array of such sensors may be processed with one or more pattern recognition algorithms such as principal component analysis (PCA), clustering algorithms, and/or classification models to enable determination of at least one clinical indicator (e.g. disease state characterization) based on the VOC sensor outputs.

One particularly advantageous example of a cross-reactive sensor is a metal oxide (MOX) sensor. Metal oxide sensors are configured to exhibit change in electrical resistance when exposed to various VOCs. The sensor consists of a metal oxide semiconductor layer on a substrate, typically heated to a high temperature using a heating element during operation. When VOCs interact with the sensor surface, they cause oxidation or reduction reactions, altering the sensor conductivity. The change in conductivity is proportional to the concentration of VOCs present, allowing for quantitative measurements.

However, MOX sensors represent just one advantageous example. Any sensor capable of broad reactivity to multiple VOCs may be utilized to implement this option. Other examples include for example conducting polymer sensors, which change their electrical properties when exposed to VOCs or optical sensors, utilizing light absorption or fluorescence to detect VOCs.

In some embodiments, the VOC measurement dataset comprises measurements of any one or more of the following VOCs: Styrene, 1 2-dimethylbenzene, Ethanol, 2-propanol, 1-propanol, 2,3-pentanedione, Benzaldehyde, Ethyl acetate.

As noted above, embodiments of the invention involve obtaining an emotional state indicator pertaining to the subject.

Regarding the emotional state indicator, in some embodiments, this may be derived based on measurements of one or more biosignals such as one or more of: heart rate variability (HRV), pulse rate variability (PRV), galvanic skin response (GSR), electroencephalography (EEG) measurements, blood pressure, respiratory rate, cortisol levels, and pupil dilation. These physiological parameters have been shown to correlate with the emotional state of an individual. By way of further example, an emotional state indicator may be derived based on other approaches such as facial expression analysis, voice analysis for emotional content, and self-reported mood scales.

In some embodiments, the emotional state indicator comprises an emotional stress measure. This measure may be derived from one or more physiological parameters, preferably one or more non-invasively measurable physiological parameters. For example, a high emotional stress state is typically characterized by increased heart rate, decreased HRV, increased GSR, and changes in EEG patterns. Stress is of particular interest due to its potential impact on VOC production and overall health status. For example, in the case that the emotional state indicator comprises an emotional stress measure, the emotional state indicator may comprise a stress state classification selected from a set of stress state classifications such as high stress and low stress, or high stress, medium stress, and low stress.

For example, in some embodiments, the obtaining the emotional state indicator comprises sensing at least one biosignal correlated with an emotional stress state of a subject, and applying a stress state classifier to the measured biosignal. The resulting emotional state indicator comprises an emotional state classification.

By way of example, the biosignal may comprise any one or more of: heart rate variability (HRV), pulse rate variability (PRV), galvanic skin response (GSR), or electroencephalography (EEG) data. Numerous studies have demonstrated the efficacy of these physiological parameters in stress detection. PRV and GSR may be particularly advantageous due to the availability of small, lightweight sensing solutions for both parameters in the current state of the art.

PRV can be measured using a photoplethysmography (PPG) sensor and is a reliable surrogate for HRV. Alternatively, HRV can be directly measured with a 2-lead ECG, which may for example be provided in a miniaturized form factor and embedded in the personal care device or a separate auxiliary device such as a wearable device.

GSR sensing devices can be provided in a small form factor. A GSR sensing device may be integrated in the personal care device, e.g. a powered toothbrush or in an auxiliary device. It is noted that if integrated in an oral care device such as a powered toothbrush, dry electrodes may be utilized. Motion compensation functionality may be provided for correcting GSR motion artefacts arising from movement of the personal care device. For example, an output of an inertial measurement unit (IMU) of the personal care device may be used for motion compensation.

Regarding the stress state classifier, this may be implemented using a pre-determined classifier model. This may comprise a trained machine learning model in some embodiments, or alternatively a rule-based algorithm. Using a machine learning model, the model may be trained using existing clinical datasets such as SWELL-KW (SWELL-Knowledge Work dataset) or WESAD (Wearable Stress and Affect Detection). Suitable machine learning model types include deep learning (DL) models such as convolutional neural networks (CNN), k-nearest neighbors (KNN), support vector machines (SVM), multilayer perceptrons (MLP), naive Bayes, or any other suitable approach.

Instead of using machine learning, a rule-based algorithm may be implemented. For example, the obtaining the stress state indicator may comprise monitoring values of one or more biosignals and detecting when one or more pre-determined biosignal criteria are met, for example the biosignal value falling within one or more pre-determined ranges, e.g. outlier ranges. For example, a calibration operation might be performed in which a baseline measure of one or more biosignals is obtained corresponding for instance to a known stress level of the subject, and wherein thresholds or ranges may be determined based on the threshold which correspond to different stress state classifications.

In some embodiments, the biosignal measurement part of the stress sensing system 60 may be integrated into the personal care device 40 or may be integrated into a separate auxiliary device, for example a wearable device, e.g. a smartwatch or fitness tracker. The separate auxiliary device may be operatively coupled with the processing device 32 to enable transfer of biosignal sensing data. In some embodiments, the processing module part may be provided by the processing device 32, e.g. a software model comprised by the processing device 32.

Instead of using biosignal measurements to determine the emotional state indicator, in some embodiments, the obtaining the emotional state measure may be realized based on the VOC measurement dataset itself.

For example, a preliminary VOC measurement dataset might be obtained using the VOC sensing subsystem 50 and an emotional state classifier applied to the preliminary VOC measurement dataset to obtain an emotional state indicator, and the analyzer 70 configured using this. Following this, subsequent VOC measurement data may be obtained and processed using the analyzer 70.

By way of example, emotional state classifier could be implemented by using a trained machine learning model, for example comprising an artificial neural network. A training dataset may be compiled comprising training data entries, each training data entry comprising a VOC measurement dataset collected for a subject, paired with a known emotional state indicator value for the subject when the measurement was obtained (e.g. a stress state indicator). The emotional state could be ascertained during measurement for example using any of a variety of established methods such as cortisol measurements, heart rate variability analysis, and/or psychological questionnaires. This training dataset may be used as the training data for training a classification model to associate newly measured VOC profiles with emotional state.

The model architecture for the emotional state classifier may comprise a deep neural network. The input to the model may comprise the VOC measurement dataset, potentially after some preprocessing such as normalization or dimensionality reduction. The output may comprise a predicted emotional state indicator, which may for example be a binary classification (e.g. high stress vs. low stress) or a more granular multi-class classification representing different emotional states, e.g. different levels of stress.

During training of the model using the training dataset, the model would learn to identify patterns in VOC concentrations that correlate with different emotional states. This might include changes in the relative concentrations of certain VOCs, the appearance or disappearance of specific compounds, or more complex interactions between multiple VOCs. The model may be trained using techniques such as backpropagation and stochastic gradient descent to minimize the difference between predictions and the ground truth stress state labels in the training data.

Once trained, the stress classification model may be deployed as part of the overall system. When a subject uses the system, a preliminary VOC measurement may first be taken and fed into the emotional state classification model. The resulting stress state prediction may then be used to configure the analyzer 70 for determining the at least one clinical indicator based on subsequent VOC measurements. This approach allows the system to infer the subject emotional state directly from the VOC data, without requiring additional sensors or inputs for stress measurement.

As discussed above, embodiments of the invention comprise use of an analyzer 70 configured to perform analysis of the VOC measurement dataset using one or more pre-determined analysis models.

The analyzer 70 may for example be or comprise a software module. The analyzer may for example be or comprise a computer program or routine.

The analyzer 70 comprises one or more pre-determined analysis models. As will be explained in more detail to follow, the one or more pre-determined analysis models may comprise machine learning models or rule-based models.

The output of the analyzer 70 is a value for at least one clinical indicator.

In some embodiments, the at least one clinical indicator may comprise a disease state classifier indicative of a predicted current or future disease state of the subject or a current progress stage of a disease. For example, in some embodiments, the at least one clinical indicator may comprise a classifier of an extant or future disease state of the subject with respect to any one or more of the following diseases: chronic kidney disease (CKD), chronic liver disease (CLD), lung cancer, breast cancer, colorectal cancer, cystic fibrosis, diabetes, lung cancer, squamous cell cancer, chronic obstructive pulmonary disease COPD, COVID-19, asthma, or sleep apnea.

In some embodiments, the at least one clinical indicator comprises a predicted concentration of a biomarker. For example, it may comprise a predicted stress-adjusted or stress-calibrated value of a biomarker. In some embodiments, it may comprise a predicted stress-adjusted or stress-calibrated value of at least one VOC included in the VOC measurement dataset. For example, the VOC measurement dataset may include a measurement value of a first VOC as having a value x. The emotional state indicator may indicate a current high stress level for the subject. The analyzer night apply a high stress analysis model which may output a corrected or stress-adjusted value of the VOC, y, which represents a 'true' level of the VOC for example corresponding to a normal (lower) stress level of the subject.

As noted above, in some embodiments, the analyzer is configured to generate a clinical indictor which corresponds to a diagnostic indicator, for example a disease state classification. In some embodiments, the one or more analysis models of the analyzer may employ an e-nose disease detection approach.

The use of an e-nose for disease detection has been documented in the literature. The general principle relies upon use of an array of multiple MOX sensors and/or electrochemical sensors. These sensors are designed to react differently to varying concentrations of VOCs. VOC measurement samples for a given one or more body-produced substances, such as breath, may be collected from both healthy and diseased subjects. Dimensionality reduction techniques, such as principal component analysis (PCA), may be applied to the set of sensor outputs of the array of sensors, to identify the primary contributing factors to variance in the data. In principal component space, clustering may be performed to distinguish between healthy and diseased populations. When samples are collected from new subjects, PCA scores are extracted from the resulting data. Based on the extracted scores, the disease state is classified.

Thus, in some embodiments, the VOC measurement dataset comprises data for each of a plurality of VOC measurement channels. Each VOC measurement channel may correspond to a sensing output of a different one of a plurality of VOC sensors. In some embodiments, each of the plurality of different VOC sensors may be configured to detect a different individual VOC. However, in more preferred embodiments, each of the plurality of VOC sensors may be a cross-reactive sensor configured to generate a sensor output associated with a mix of VOC gases in the environment. Each VOC sensor in the plurality of VOC sensors may be configured with a different sensitivity or different selectivity to different VOCs. The result is that the output of the plurality of VOC sensors is a multi-dimensional VOC measurement dataset comprising a plurality of VOC measurement channels (for example one channel corresponding to each sensor).

By way of example, each of the plurality of VOC sensors may comprise a metal oxide (MOX) sensor, and wherein the plurality of MOX sensors care configured to vary in their respective sensitivity and reactivity to different VOC mixtures. This can be achieved by varying one or more of the material composition, surface treatment and/or operating conditions of the different sensors. For example, different metal oxides, such as tin oxide, zinc oxide, or tungsten oxide, have different sensitivity to various volatile organic compounds (VOCs). Furthermore, doping these materials with catalytic metals, such as platinum or palladium, further alters chemical sensitivity. Furthermore, operating identical materials at different temperatures changes reaction kinetics at the sensor surface, leading to varied sensor responses. As a result, each MOX sensor in the array may generate a unique signal profile when exposed to the same gas mixture. The resulting diversity in output across the sensor array creates a multi-dimensional dataset, which can then be analyzed to detect and classify disease states.

Analysis of the multi-dimensional VOC measurement dataset may be achieved using a dimensionality reduction approach in which the plurality of measurement channels sampled using the VOC sensing subsystem is reduced to a smaller number of measurement channels, where these may correspond to pre-determined principal components.

By way of example, in some embodiments. the analysis performed by the analyzer comprises transforming the measured VOC dataset into a reduced dimensionality dataset comprising a reduced number of channels. The reduced channels might be referred to as 'synthetic channels'. Each of the channels of the reduced dimensionality dataset may be formed as a function of two or more of the VOC measurement channels. For example, each of the channels of the reduced dimensionality dataset may comprise a linear combination of two or more of the VOC measurement channels.

For example, the reduced dimensionality dataset may comprise a plurality of synthetic channels, wherein the plurality of synthetic channels is smaller in number than the plurality of VOC measurement channels, and wherein each of the synthetic channels is a linear combination of two or more of the VOC measurement channels.

As noted above, the channels of the reduced dimensionality dataset may correspond to a pre-determined set of Principal Components of the plurality of VOC measurement channels.

For example, principal component analysis (PCA) can be applied to the VOC measurement channels in advance in order to determine a reduced number of principal components which are each formed from a linear combination of a subset of the VOC measurement channels. For example, VOC measurement samples for a given one or more body-produced substances, such as breath, may be collected from both healthy and diseased subjects. Principal component analysis (PCA) may be applied to identify the primary contributing factors to variance in the data with regards to the value of the clinical indicator of interest. In principal component space, clustering may be performed to distinguish between different sub-populations associated with different values of the clinical indicator of interest. During operation of the method, the measured plurality of VOC channels can be processed to compute the pre-determined principal component channels, and wherein the one or more models of the analyzer are configured to map between these principal component channels and the at least one clinical indicator.

The analysis performed by the analyzer may thus in some embodiments comprise mapping values for the reduced channels of the reduced dimensionality dataset to at least one clinical indicator.

As discussed above, the method of analysis employed by the analyzer is configurable in dependence upon the emotional state of the user or subject.

In general terms, this means that the way that the analysis is performed varies in dependence upon the emotional state of the subject.

For example, one way of describing this is that the analyzer may be understood as being configured to use the one or more models to perform mapping of VOC measurement data comprised by the VOC measurement dataset to at least one clinical indicator, and wherein the calibration of the mapping is configured in dependence upon the emotional state. In other words, a calibration of the analyzer is adjusted in dependence upon the emotional state.

This can be achieved either by using different models in the case of different emotional states or by tuning parameters of the model in dependence upon the emotional state. Changing the calibration of the analysis for example entails adjusting an association between a VOC measurement profile of a given subject and a predicted clinical indicator. Different VOC measurement profiles are associated with each disease state classification dependent upon the emotional state of the user. The adjusting the calibration of the analysis comprises changing the association between the VOC measurements and the disease state classifications.

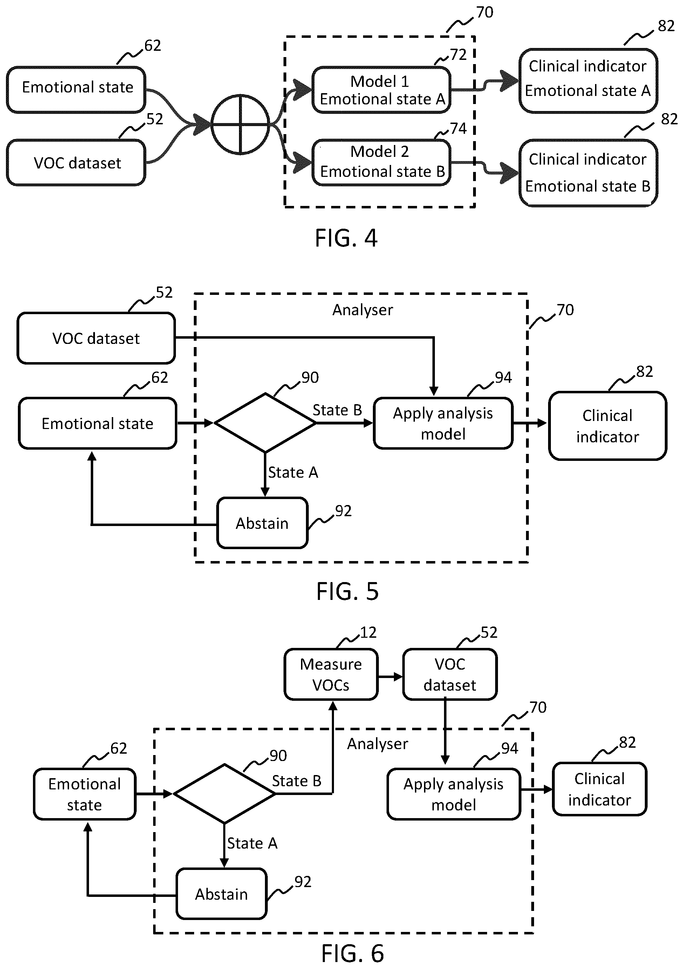

With regard to the configuring of the analysis performed by the analyzer responsive to the emotional state measure, there are a multitude of different ways to implement this. A non-exhaustive set of different possible implementations will now be discussed, with reference to FIG. 4 to FIG. 6

One simple approach is to provide an analyzer comprising a bank of different analysis models, and wherein the analyzer employs a different subset of one or more of the bank of different analysis models depending upon the emotional state of the subject.

An example according to this approach is schematically illustrated in FIG. 4.

In the example of FIG, 4, the analyzer 70 comprises two models: a first model 72 and a second model 74. The first model 72 is configured for use when the emotional state of the subject is a first emotional state (labelled emotional state A in FIG. 4) and the second model 74 is configured for use when the emotional state of the subject is a second emotional state (labelled emotional state B in FIG. 4). Each of the first 72 and second models 74 is configured to output a value of a same at least one clinical indicator 82 responsive to an input dataset derived from a VOC measurement dataset 52. Responsive to an input indicative of the emotional state indicator 62 of the subject, the analyzer 70 selectively employs one of the first 72 and second 74 analysis models to process the VOC measurement dataset 52.

By way of one example implementation, the first analysis model 72 may correspond to a higher stress model for use when an emotional state of the subject corresponds to a high emotional stress state and a lower stress model 74 when the emotional state indicator of the subject corresponds to a lower stress state. By way of example, the higher stress model is configured for use when subject stress level is above a threshold and the lower stress model is configured for use when the subject stress level is below a threshold.

The first 72 and second 74 models may in some embodiments each comprise a machine learning model, for example comprising one or more artificial neural networks.

In one set of embodiments, each of the two analysis models 72, 74 may be provided as a disease classification model for determining a disease state of the individual. Each model may use an "e-nose" analysis method. Each model may be trained based on training data collected from both diseased and healthy individuals in stressed and unstressed states. Standard protocols for inducing psychological stress in experimental environments, which are well-documented in the literature, may be employed to elicit stress responses during the collection of training VOC data. One such protocol that may be utilized is the Trier Social Stress Test (TSST). The stress state of individuals during data collection may be confirmed through the measurement of vital signs.

This strategy effectively results in the creation of two distinct clinical indicator classification models, which can be selectively employed based on the measured emotional state of the user. The first model 72 is calibrated for use with data collected from subjects in a stressed state, while the second model 74 is calibrated for use with data collected from subjects in an unstressed state. The appropriate model is selected based on the emotional state indicator derived from the user's physiological measurements.

This approach offers the advantage of simplicity. There is no need for user-dependent calibration. There is also no need, for example, to train a model having a parameter which is dependent upon the emotional state of the user. Instead, individual discrete models attuned to different emotional states can be deployed.

Although in the example discussed above, the two analysis models 72, 74 were configured for different emotional stress states, it is to be understood that the same principle can be applied for a different category of emotional state. Furthermore, although in the example above each model outputs a disease classification, the models may be trained to output a different clinical indicator, for example a predicted biomarker value.

Furthermore, although in the example presented in FIG. 4, the analyzer 70 comprises a set of two analysis models, as a more general principle, the analyzer 70 may comprise any number, N, of analysis models corresponding to varying levels of measured stress. For example, the analyzer 70 may comprise a set of three or more different analysis models, each corresponding to a different gradation of emotional stress level, and wherein configuring the method of analysis performed by the analyzer 70 comprises selecting one of the models to employ in dependence upon a gradation of stress level indicated by the emotional state indicator.

In other words, there may be provided a plurality of different stress-dependent models, each applicable responsive to a different level of emotional stress of the subject. In this embodiment, it is assumed that the number of disease classification models are equal to the number of classification states of emotional stress.

In some embodiments, the analyzer 70 may be configured to abstain from performing the analysis responsive to the emotional state indicator 62 meeting at least one pre-determined condition. For example, in a high stress state, the analyzer abstains from generating the clinical indicator because reliability would be low in the high stress state.

In other words, the system may be configured to exclude or flag measurements that are taken under particular emotional state conditions (e.g. high emotional stress) and prevent them from being included in progressive or cumulative analysis of disease.

An example implementation is shown in FIG. 5. The analyzer 70 is configured to perform an assessment 90 of the emotional state indicator 62 comprising determining whether the emotional state indicator meets at least one predetermined condition. The pre-determined condition may correspond to a particular emotional state classification. For example, the analyzer may be configured to classify the received emotional state indicator 62 into one of a set of emotional state classifications. In the example of FIG. 5, the set of possible emotional state classifications numbers two: emotional state A and emotional state B. However, a larger number of classifications is also possible. By way of example, emotional state A may correspond to a high emotional stress state and emotional state B may correspond to a low emotional stress state. The assessment 90 comprises determining whether the emotional state indicator classification is state A or state B. If the emotional state indicator meets the pre-determined criterion of falling into the classification of emotional state A, then the analyzer 70 is configured to abstain 92 from performing 94 the analysis of the VOC measurement dataset 52. For example, the analyzer is configured to abstain from applying any of the set of one or more analysis models to the received VOC measurement dataset 52. If the assessment 90 determines that the emotional state indicator does not meet the predetermined condition (in this case that the emotional state indicator is not emotional state A, but rather is emotional state B, e.g. the subject is in a low stress state rather than a high stress state), then the analyzer 70 is configured to perform 94 the analysis of the VOC measurement dataset 52 using at least one of the one or more analysis models. The analyzer 70 is configured to output a value of the at least one clinical indicator 82 based thereon.

In a variation on the embodiment described above with reference to FIG. 6, in some embodiments, the emotional state indicator 62 is used as a gate to determine whether to obtain the VOC measurement dataset 52 by sampling the one or more VOC sensors. In this embodiment, it is proposed that, responsive to the emotional state indicator meeting a pre-determined condition (e.g. corresponding to a high emotional stress state), the method may comprise blocking or abstaining from measurement of the VOC measurement dataset and from applying analysis to determine the at least one clinical indicator. The advantage of this embodiment is that system resources are conserved by withholding from sampling the VOC sensors in the event that the emotional state of the user is not suitable for clinical indicator analysis. The system may be configured to prompt the user to reattempt measurement and analysis at a different later time, e.g. after time has elapsed (e.g. 10-30 minutes), and/or the system may monitor the emotional state indicator over time and detect when the emotional state indicator is suitable for the analysis to be performed.

An example implementation is shown schematically in FIG. 6. In this example, the analyzer 70 is configured to perform an assessment 90 comprising determining whether the emotional state indicator meets a pre-determined condition. The pre-determined condition may correspond to a particular emotional state classification. For example, the analyzer may be configured to classify the received emotional state indicator 62 into one of a set of emotional state classifications. In the example of FIG. 6, the set of possible emotional state classifications numbers two: emotional state A and emotional state B. However, a larger number of classifications is also possible. By way of example, emotional state A may correspond to a high emotional stress state and emotional state B may correspond to a low emotional stress state. The assessment 90 comprises determining whether the emotional state indicator classification is state A or state B. If the emotional state indicator meets the pre-determined criterion of falling into the classification of emotional state A, then the analyzer 70 is configured to abstain 92 from performing 94 the analysis of the VOC measurement dataset 52. The analyzer may then control a monitoring loop in which the emotional state indicator 62 is repeatedly evaluated and assessed 90 against the pre-determined condition. In other words, the analyzer 70 may further implement a polling mechanism to periodically reassess the emotional state indicator 62. This polling mechanism allows the system to continuously or intermittently re-determine the emotional state indicator 62 and to assess 90 whether the emotional state has changed to a condition suitable for VOC measurement and analysis. When the polling mechanism detects that the emotional state indicator has transitioned to meet the pre-determined criterion for analysis (e.g., a low stress state), the system may then proceed with obtaining 12 the VOC measurement dataset 52 and performing 94 the analysis to determine the clinical indicator 82.

In other words, in some embodiments, the method may comprise monitoring the emotional state indicator 62 over a period of time and may comprise triggering sampling of the VOC measurement dataset and performing 94 analysis of the VOC measurement dataset using at least one of the one or more analysis models responsive to detection of the emotional state indicator 62 changing from a state of not meeting the at least one pre-determined condition to a state of meeting the at least one pre-determined condition (or vice versa).

Instead of providing a bank of different analysis models, each pre-configured for application under different emotional state conditions, an alternative implementation approach is to provide at least one analysis model wherein the emotional state is a variable parameter of the model, or where the emotional state is an input to the model. In this set of embodiments, the analysis model employed by the analyzer would require both a VOC measurement dataset and an emotional state indicator (e.g. stress measurement) as inputs, both obtained concurrently in time. This model may offer improved accuracy since the configuration of the analysis dependent upon the emotional state of the subject can be more finely tuned.

Thus, in accordance with this set of embodiments, it is proposed to provide the analyzer 70 wherein at least one of the one or more analysis models used by the analyzer comprises one or more model parameters which are configured in dependence upon the emotional state indicator.

In other words, a mapping between the VOC measurement profile and the clinical indicator, as represented by one or more parameters of the model, is adjusted depending on the emotional state, e.g. stress level, of the subject.

In some examples, the emotional state indicator may form an input to one or more analysis models. In other words, the analyzer may comprise at least one analysis model for which both emotional state and VOC measurement data form inputs to the model.

To implement this, in some embodiments, the analyzer may comprise at least one analysis model which comprises a multi-input neural network. This type of architecture allows for the integration of different types of data in a single model. This may enable the model to learn relationships between VOCs, emotional state, and the at least one clinical indicator.

The VOC measurement data may be processed by one branch of the neural network. This branch may comprise a plurality of convolutional layers for extracting relevant features from the VOC measurement dataset, followed by pooling layers to reduce dimensionality. A separate branch of the network may handle the emotional state indicator. By way of non-limiting example, this branch may comprise one or more dense layers.

The outputs from the two branches may then be combined, for example through concatenation or a different fusion mechanism. The combined representation may be fed through several dense layers, gradually reducing in size, and subsequently a final output layer which outputs the clinical indicator prediction.

The entire network may be trained end-to-end, allowing it to learn the optimal way to integrate the emotional state information when analyzing the VOC data.

Regarding training, a suitable training dataset may be compiled comprising a plurality of training data entries, each comprising a VOC measurement dataset for subject, an emotional state indicator corresponding to an emotional state of the subject at the time of acquiring the VOC measurement dataset, and a ground truth value of the at least one clinical indicator for the subject. This training data should cover a wide range of emotional states and subject clinical conditions to ensure the model can generalize well. The training process comprises feeding the training data through the network, comparing the predicted values of the at least one clinical indicator with the true values, and adjusting the network parameters using backpropagation to minimize the prediction error.

During operation, new VOC measurements from the VOC measurement dataset and a concurrently acquired emotional state indicator may be fed into the respective branches of the trained network. The network accordingly outputs a clinical indicator prediction that takes into account both the VOC profile and the subject emotional state.

In all embodiments, it should be noted that measurement interval and analysis window duration may vary. For example, emotional state indicator measurements and VOC measurements can be analyzed at a single point in time or could be analyzed longitudinally over long periods of time (days, weeks, months, years). This may allow for capturing variations in stress or clinical state within a single day (e.g. stress levels/cortisol peak in the morning and taper throughout the day) or progression (e.g. improvement) over weeks.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A processing device (32) comprising one or more processors (36) configured to perform a method (10) for determining at least one clinical indicator (82) for a subject based on volatile organic compound, VOC, measurements, the method comprising:
obtaining (12) a VOC measurement dataset (52) for the subject indicative of measurement of one or more VOCs in one or more body-produced substances of the subject;
obtaining (14) an emotional state indicator (62) for the subject, indicative of at least one characteristic of an emotional state of the subject;
applying (16) an analyzer (70) configured to perform analysis of the VOC measurement dataset using one or more pre-determined analysis models (72, 74) and output at least one clinical indicator (82) responsive thereto;
wherein a method of analysis applied by the analyzer using the one or more models is configurable, and wherein the method of analysis is configured (18) in dependence upon the emotional state of the subject.

2. The processing device (32) of claim 1, wherein the analyzer (70) comprises a bank of different analysis models (72, 74), and wherein the analyzer is configured to employ a different subset of one or more of the analysis models depending upon the emotional state (62).

3. The processing device (32) of claim 1 or 2,
wherein the emotional state indicator comprises an emotional stress measure; and
optionally wherein obtaining the emotional stress indicator comprises obtaining one or more of: a heart rate variability (HRV) measure, a pulse rate variability (PRV) measure, a galvanic skin response (GSR) measure, an electrocardiogram (ECG) measurement, an electroencephalography (EEG) measurement, a body temperature measurement, and a blood pressure measurement.

4. The processing device (32) of claim 3, wherein the analyzer (70) comprises a higher stress model (72) and lower stress model (74) and is configured to use the higher stress model (72) when the emotional state indicator (62) corresponds to a high stress state (A) and the lower stress model (74) when the emotional state indicator (62) corresponds to a low stress state (B).

5. The processing device (32) of claim 3 or 4, wherein the analyzer (70) comprises three or more different analysis models, each corresponding to a different gradation of emotional stress level and wherein configuring the method of analysis comprises selecting one of the analysis models to employ in dependence upon a gradation of emotional stress level indicated by the emotional state indicator.

6. The processing device (32) of any preceding claim, wherein the analyzer (70) is configured to abstain (92) from performing (94) the analysis responsive to the emotional state indicator (62) meeting at least one pre-determined condition.

7. The processing device (32) of any preceding claim, wherein at least one of the one or more analysis models used by the analyzer (70) comprises one or more model parameters which are configured in dependence upon the emotional state indicator (62).

8. The processing device (32) of any preceding claim, wherein the obtaining (12) the VOC measurement dataset (52) comprises using a non-invasive VOC sensing subsystem to obtain the VOC measurement dataset (52).

9. The processing device (32) of any preceding claim, wherein the obtaining (12) the VOC measurement dataset (52) comprises obtaining the VOC measurement dataset using a VOC sensing subsystem (50) comprising one or more VOC sensors integrated in a personal care device (40), and optionally wherein the personal care device is an oral care device.

10. The processing device (32) of any preceding claim,
wherein the VOC measurement dataset (52) comprises data for each of a plurality of VOC measurement channels, and wherein the analysis performed by the analyzer (70) comprises transforming the measured VOC dataset into a reduced dimensionality dataset comprising a reduced number of channels, and
optionally wherein the channels of the reduced dimensionality dataset correspond to a pre-determined set of Principal Components of the plurality of VOC measurement channels.

11. The processing device (32) of any preceding claim, wherein the at least one clinical indicator comprises a disease state classifier indicative of a predicted current or future disease state of the subject or a current progress stage of a disease.

12. A system (30), comprising:
a personal care device (40);
a VOC sensing subsystem (50) integrated in the personal care device (40), comprising one or more VOC sensors arranged to be non-invasively exposed to at least one bodily-produced substance, such as at least one body liquid or gas during operation of the personal care device; and
a processing device (32) in accordance with any of claims 1-11 operatively coupled with the VOC sensing subsystem (50) and configured to obtain the VOC measurement dataset using the VOC sensing subsystem (50).

13. The system of claim 12,
wherein the VOC sensing subsystem (50) comprises:
at least one VOC sensor integrated in a mouth-receivable portion of the device for sensing concentration of at least one VOC in saliva of the subject; and/or
at least one VOC sensor arranged for sensing concentration of at least one VOC in exhaled breath during use of the personal care device; and/or
at least one VOC sensor integrated in a handle portion of the device arranged for sensing concentration of at least one VOC in sweat of the subject during use of the device.

14. A method (10) for determining at least one clinical indicator for a subject based on volatile organic compound, VOC, measurements, the method comprising:
obtaining (12) a VOC measurement dataset (52) for the subject indicative of measurement of one or more VOCs in one or more body-produced substances of the subject;
obtaining (14) an emotional state indicator (62) for the subject, indicative of at least one characteristic of an emotional state of the subject;
applying (16) an analyzer (70) configured to perform analysis of the VOC measurement dataset (52) using one or more pre-determined analysis models and output at least one clinical indicator responsive thereto;
wherein a method of analysis applied by the analyzer using the one or more models is configurable, and wherein the method of analysis is configured (18) in dependence upon the emotional state of the subject.

15. A computer program product comprising computer program code configured, when run on a processor, to cause the processor to perform a method (10) for determining at least one clinical indicator (82) for a subject based on volatile organic compound, VOC, measurements, the method comprising:
obtaining (12) a VOC measurement dataset (52) for the subject indicative of measurement of one or more VOCs in one or more body-produced substances of the subject;
obtaining (14) an emotional state indicator (62) for the subject, indicative of at least one characteristic of an emotional state of the subject;
applying (16) an analyzer (70) configured to perform analysis of the VOC measurement dataset using one or more pre-determined analysis models (72, 74) and output at least one clinical indicator (82) responsive thereto;
wherein a method of analysis applied by the analyzer using the one or more models is configurable, and wherein the method of analysis is configured (18) in dependence upon the emotional state of the subject.
